(19) Europäisches Patentamt
European Patent Office
Office européen des brevets

(11) **EP 4 576 114 A1**

(12) **EUROPEAN PATENT APPLICATION**

(43) Date of publication:
**25.06.2025 Bulletin 2025/26**

(21) Application number: **23290047.2**

(22) Date of filing: **20.12.2023**

(51) International Patent Classification (IPC):
***G16H 40/40*** (2018.01) ***G01N 35/00*** (2006.01)

(52) Cooperative Patent Classification (CPC):
**G16H 40/40; G01N 35/00603; G01N 35/00623**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Beckman Coulter, Inc.
Brea, CA 92821 (US)**

(72) Inventor: **Varlet, Eric
59790 Ronchin (FR)**

(74) Representative: **Maiwald GmbH
Elisenhof
Elisenstraße 3
80335 München (DE)**

(54) **METHOD, DEVICE AND SYSTEM FOR TESTING BIOLOGICAL SAMPLES IN CASE OF FAILURE DETECTION**

(57)    Summarizing the invention, a method is provided. The method comprises determining, by a computing device, a failure of a laboratory instrument configured to carry out clinical tests on biological samples; accessing, by the computing device, test data comprising information specifying: a plurality of clinical tests that were carried out by the laboratory instrument and that may have been affected by the failure of the laboratory instrument, a chronological sequence of the plurality of clinical tests, and a respective plurality of test results associated with the plurality of clinical tests; identifying, by the computing device, a reference clinical test among the plurality of clinical tests, the reference clinical test being a clinical test that has not been affected by the failure of the laboratory instrument; determining, by the computing device, a proper subset of clinical tests of the plurality of clinical tests based on the reference clinical test; causing, by the computing device, the proper subset of clinical tests to be rerun.

Figure 1

## Description

<u>Technical Field</u>

The following description relates to the field of testing of biological samples.

<u>Background</u>

Analytic and clinical laboratories typically comprise laboratory instruments that are configured to automatically carry out one or more clinical tests (e.g. clinical chemistry and/or immunoassay tests) on biological samples (such as blood samples).

Quality control ensures the accuracy, reliability, and precision of clinical test results produced by the analytic and clinical laboratories. In particular, quality control has the goal of identifying and correcting any errors or variations in the testing process, ultimately minimizing the risk of producing incorrect or misleading results. Typically, among other measures, quality control checks are regularly performed on a laboratory instrument that carries out the clinical tests.

Conventionally, if a failure of the laboratory instrument is detected during a quality control check, all the clinical tests that have been carried out after a previous quality control check (that had not identified any failure) are rerun.

<u>Summary</u>

It is an object of the invention to increase the efficiency of sample testing by laboratory instruments in case of failure detection, in particular time-wise as well as by reducing reagent and/or sample consumption.

The achievement of this object in accordance with the invention is set out in the independent claims. Further developments of the invention are the subject matter of the dependent claims.

According to a first aspect, a method, e.g. a computer-implemented method, is provided. The method comprises:

- determining, by a computing device, a failure of a laboratory instrument configured to carry out clinical tests on biological samples;
- accessing, by the computing device, test data comprising information specifying:

  -- a plurality of clinical tests that were carried out by the laboratory instrument and that may have been affected by the failure of the laboratory instrument,
  -- a chronological sequence of the plurality of clinical tests, and
  -- a respective plurality of test results associated with the plurality of clinical tests;

- identifying, by the computing device, a reference clinical test among the plurality of clinical tests, the reference clinical test being a clinical test that has not been affected by the failure of the laboratory instrument;
- determining, by the computing device, a proper subset of clinical tests of the plurality of clinical tests based on the reference clinical test;
- causing, by the computing device, the proper subset of clinical tests to be rerun.

According to the present invention, a computing device, e.g. the computing device carrying out the method, may comprise at least one memory and at least one processor, exemplarily a plurality of processors and/or a plurality of memories. A computing device may also comprise one or more input/output units. According to the present invention, a computing device may be a distributed computing system, e.g. a computing network. For example, the computing device may comprise a computing device integrated with the laboratory instrument and another computing device remote from the computing device integrated with the laboratory instrument. In another example, the computing device may comprise one or more computing devices integrated with the laboratory instrument.

The laboratory instrument is configured to carry out one or more clinical tests. A clinical test may comprise one or more procedures that, when carried out on a biological sample, allow for estimating the value of a parameter, e.g. a clinical parameter. Accordingly, each clinical test is associated with a test result, which e.g. may comprise the (estimated) value of the parameter and/or other values derived from the parameter value. In particular, a clinical test may comprise physical, biological, optical, mechanical, immunological, and/or chemical procedures. Exemplarily, a clinical test may be an immunoassay test or a chemical test.

The biological sample (or simply "sample") may be a sample of a bodily fluid of a human or animal subject. For example, the bodily fluid may be a physiological fluid, such as blood, saliva, urine, sweat, amniotic fluid, cerebrospinal fluid, ascites fluid, or the like. The biological sample may be put into a sample container after collection and it may be held in the container while being processed. The sample container may be a sample tube. Typically, sample tubes comprise a closed tube end and an end opposite thereto. The latter end defines an opening for inserting the sample in the sample tube. The opening may be closed, e.g. sealed, by a cap.

The laboratory instrument may comprise at least one analyser (i.e. one or more analysers) configured to carry out one or more clinical tests. Each analyser may comprise an aspiration unit configured to aspirate a volume of sample from the sample container and then dispense it, as well as an analytical unit configured to carry out the one or more clinical tests on said volume.

A failure of the laboratory instrument is a deviation from the correct functioning of the laboratory instrument. The failure may be identified as a non-compliance with positive criteria and/or as an anomaly satisfying negative criteria. In particular, the positive criteria may define a correct/normal state and/or functioning of the laboratory instrument and the negative criteria may define an incorrect/abnormal state and/or functioning of the laboratory instrument. The failure of the laboratory instrument affects the performance of the clinical tests. In particular, the test results produced by a faulty laboratory instrument may be incorrect, in that the parameter value as estimated by the laboratory instrument does not correspond to the actual parameter value.

For instance, the cause of the failure of the instrument may be at least one of the following: a faulty calibration of the laboratory instrument, a mechanical malfunction of the laboratory instrument, a contamination of the laboratory instrument and/or of a reagent, a software malfunction of the laboratory instrument, an erroneous preparation of a reagent, external conditions that affect the performance of the laboratory instrument, e.g. a room temperature outside a range defined for normal operation conditions.

Determining the failure of the laboratory instrument is equivalent to determining that a failure of the laboratory instrument has occurred. Exemplarily, determining the failure of the laboratory instrument may comprise using control result data. In particular, the computing device may evaluate the control result data against positive and/or negative criteria, as discussed above. If the control result data do not satisfy the positive criteria and/or satisfy the negative criteria, the failure of the laboratory instrument may be determined.

The control result data may comprise information specifying at least one result of at least one control test. A control test may be a clinical test carried out on a control material for which the value of the parameter (e.g. the clinical parameter) to be estimated is already known. For instance, the control material may mimic a biological sample (e.g. blood), such as with regards to physical, biological, optical and/or chemical properties. Positive criteria in this case may comprise a condition that the result of the control test be compatible with the actual value, i.e. the known parameter value, e.g. that the result of the control test be within a numerical range including (e.g. centred on) the known parameter value. In other words, it may be checked whether the laboratory instrument provides an expected result within some tolerance limits. For instance, the control material may be associated with a control result and a standard deviation and the result of the control test may be considered compatible with the control result if it differs from the control result by not more than one or two standard deviations.

Exemplarily, determining the failure of the laboratory instrument may further comprise causing, by the computing device, the laboratory instrument to perform the at least one control test. In the present disclosure, "causing, by the computing device, the laboratory instrument to do something" may comprise instructing, by the computing device, the laboratory instrument to do something. In this case, the computing device may be external to the laboratory instrument. In another example, "causing, by the computing device, the laboratory instrument to do something" may comprise controlling, by the computing device, one or more components of the laboratory instrument to do something. In this case, the computing device may be internal to the laboratory instrument and may control other components of the laboratory instrument so that something is done.

Performing at least one control test may be part of a quality control routine. Exemplarily, a quality control routine may be initiated when a trigger event occurs. The trigger event may e.g. be the fact that a certain number of clinical tests have been performed or that a certain amount of time has elapsed since the last quality control routine was performed or since the operations of the laboratory instrument have started. In other examples, the trigger event may be that an average, in particular a moving average (e.g. the exponentially weighted moving average - EWMA) of a predetermined set of test results of the clinical tests performed by the laboratory instrument lies outside of a predefined range.

The flow of a quality control routine may be as follows:

- determining, by the computing device, that a trigger event has occurred;
- notifying a user, by the computing device, that the trigger has occurred;
- making, by the user, a container containing the control material available to the laboratory instrument (e.g. loading the container in the laboratory instrument or into an input unit or a transport unit connected to the laboratory instrument);
- causing, by the computing device, the laboratory instrument to perform at least one control test on the control material to obtain control result data;
- determining, by the computing device, whether a failure of the laboratory instrument has occurred by evaluating the control result data against positive and/or negative criteria.

In other cases, the quality control routine may be completely automated, i.e. without the intervention of a user. For instance, a plurality of containers containing control materials may be available to automated transport means (e.g. a gripper or a conveyor) for being loaded in the laboratory instrument.

In another example, determining the failure of the laboratory instrument may comprise accessing, by the computing device, failure data relative to the laboratory instrument, the failure data comprising information that a failure of the laboratory instrument has occurred. For instance, the failure data may comprise a binary flag that is set to a value (e.g. 0) when there is no failure and to the other value (e.g. 1) when there is a failure.

In the present disclosure, "accessing data" (such as the failure data) may comprise retrieving the data e.g. from the at least one memory of the computing device that carries out the method, from the memory of another computing device, or from another remote data storage (a database, a secondary memory, a cloud storage or the like). Additionally or alternatively, "accessing data" may comprise receiving the data, e.g. from a user or a computing device different from the computing device accessing the data. The two options are not mutually exclusive. For instance, accessing data may comprise receiving the data, storing the data in the memory of the computer device and retrieving the data by accessing said memory.

The moment in time at which the computing device determines the failure of the laboratory instrument may be referred to as "failure determination timepoint".

The method further comprises accessing the test data. In particular, the step of accessing the test data may be performed as a consequence of determining the failure of the laboratory instrument. In particular, if the moment in time at which the test data are accessed is denoted as "access timepoint", the access timepoint is subsequent to the failure determination timepoint.

The test data comprise information relative to the plurality of clinical tests that were carried out by the laboratory instrument and that may have been affected by the failure of the laboratory instrument. Such a plurality of clinical tests may also be referred to as "the set of potentially affected clinical tests". In particular, a potentially affected clinical test is a clinical test that was performed by the laboratory instrument of which the failure has been determined and for which it cannot be excluded that the failure has impacted the procedure. More particularly, the potentially affected clinical tests have a non-zero probability of having been affected by the failure of the laboratory instrument.

Accessing the test data may comprise selecting the plurality of clinical tests (e.g. out of a totality of clinical tests carried out by the laboratory instrument) based on one or more constraints. In other words, the set of potentially affected clinical tests may be selected by using one or more constraints.

In one example, the set of potentially affected clinical tests may comprise tests carried out by the laboratory instrument that satisfy one or more constraints. Thus, the one or more constraints may define the property of being potentially affected by the failure.

In other words, in this example the method may comprise: accessing, by the computing device, test data comprising information specifying a plurality of clinical tests that were carried out by the laboratory instrument and that satisfy one or more constraints, the one or more constraints defining clinical tests that may have been affected by the failure of the laboratory instrument.

In another example, the one or more constraints may be used to identify one or more particular clinical tests and the plurality of clinical tests may be selected on the basis of the one or more particular clinical tests, in particular based on a relation (such as a temporal relation) with the one or more particular clinical tests.

Exemplarily, one constraint may be that the set of potentially affected clinical tests lie within a time range having a start timepoint and an end timepoint, wherein the end timepoint is the access timepoint. This may be referred to as "time range constraint". In some cases, the laboratory instrument may have carried out one or more clinical tests in between the failure determination timepoint and the access timepoint. Otherwise, the end timepoint may equivalently be the failure determination timepoint.

Accordingly, the time range may be such that the clinical tests that have been performed within said time range are those that may have been affected by the failure of the laboratory instrument. Since the end timepoint of the time range is always known (being the access timepoint), the time range may be defined by setting its duration or by setting the start timepoint.

In one example, the start timepoint may be the moment in time at which it was last determined that the laboratory instrument was functioning correctly. For instance, it may be the timepoint at which one or more control tests were performed and, based on the result(s) of the one or more control tests, it was determined that the laboratory instrument was functioning correctly, or, said otherwise, that no failure of the laboratory instrument had occurred.

In another example, the start timepoint may be determined based on the end timepoint, as in at a fixed, predetermined temporal distance from the end timepoint. The temporal distance may be expressed in absolute time (e.g. in seconds, minutes, hours) or in number of clinical tests performed.

In some cases, the time range constraint may be the only constraint, while in other cases there may be one or more additional/alternative constraints, as discussed below.

The plurality of clinical tests in the test data may all have been affected by the failure of the laboratory instrument because it is not known when the failure occurred. If the moment in time at which the failure occurred is denoted as "failure occurrence timepoint", the laboratory instrument works correctly up to the failure occurrence timepoint and does not function correctly from the failure occurrence timepoint onwards. In some cases, any clinical test that was performed before the failure occurrence timepoint was not affected by the failure and any clinical test that was performed after the failure occurrence timepoint was affected by the failure of the laboratory instrument. In particular, the failure occurrence timepoint is prior to (or substantially simultaneous with) the failure determination timepoint, but it may be at any moment between the start timepoint and the failure determination timepoint.

Further to the information specifying the set of potentially affected clinical tests, the test data comprise information specifying the chronological sequence of the plurality of clinical tests. Thus, the test data not only indicate which clinical tests may have been affected by the failure of the laboratory instrument, but in which chronological order these clinical tests have been performed, namely an ordered temporal sequence for the potentially affected clinical tests can be identified. Based on the chronological sequence, it is possible to identify, for each given clinical test, the clinical test that was performed immediately prior to it and the clinical test that was performed immediately after it. In particular, given any pair of clinical tests, it is possible to determine which clinical test was performed sooner (i.e. at an earlier timepoint) and which was performed later (i.e. at a later timepoint).

In some examples, the test data may be formatted such that the clinical tests are arranged in the chronological sequence, e.g. in a chronologically ordered list. Accordingly, the temporal information may be contained in the order in which the clinical tests are stored. In other examples, the test data may contain absolute time information about each clinical test, e.g. timestamps. The chronological sequence may then be derived from the absolute time information.

Finally, the test data comprise information specifying a respective plurality of test results associated with the plurality of clinical tests. In particular, each clinical test of the plurality of clinical tests is associated with a respective test result of the plurality of test results. The test results are those obtained when the clinical tests were carried out by the laboratory instrument. Since the clinical tests may have been affected by the failure of the laboratory instrument, the test results may be incorrect.

The method further comprises identifying, by the computing device, a reference clinical test among the plurality of clinical tests, the reference clinical test being a clinical test that has not been affected by the failure of the laboratory instrument. The reference clinical test belongs to the set of potentially affected clinical tests, but in this step it is determined that actually the reference clinical test was not affected by the failure. In particular, identifying the reference clinical test may comprise determining that a given clinical test of the plurality of clinical tests was not affected by the failure of the laboratory instrument and setting said given clinical as the reference clinical test. In other words, identifying the reference clinical test may comprise finding a clinical test that was not affected by the failure of the laboratory instrument, e.g. by searching among the set of potentially affected clinical tests until a clinical test that was not affected by the failure of the laboratory instrument is found.

In particular, the reference clinical test is a clinical test that was performed before the failure occurrence timepoint. The identification of the reference clinical test leads to a narrowing down of the time frame in which the failure occurrence timepoint lies, because the failure must have happened after the reference clinical test was performed. Thus, the reference clinical test is such that each clinical test of the plurality of clinical tests that is chronologically antecedent to the reference clinical test was not affected by the failure of the laboratory instrument, because the failure had not yet happened.

Exemplarily, the reference clinical test may be identified by performing a comparison between a comparison value and one or more reference values, wherein the comparison value is obtained from one or more test results of the plurality of test results, the one or more test results including the test result that is associated with the reference clinical test. In some cases, the comparison value may be compared with a single reference value, while in other cases the comparison value may be compared with two reference values, or more.

As discussed in more detail below, in some examples the comparison value may be the test result associated with the reference clinical test. In other examples, the comparison value may be the exponentially weighted moving average obtained from a subgroup of the plurality of test results including the test result associated with the reference clinical test and the test results of some or all the clinical tests chronologically preceding it.

In particular, the one or more reference values are values indicative of a proper functioning of the laboratory instrument. Accordingly, the comparison between the comparison value and the one or more reference values provides an insight into the functioning of the laboratory instrument around the timepoint at which the reference clinical test was performed.

Exemplarily, performing the comparison may comprise computing a difference between the comparison value and (each of) the reference value(s) and identifying the reference clinical test may further comprise checking whether the difference(s) meet one or more requirements. For instance, a requirement may be any one of the following: the difference is greater than a certain threshold, the difference is a negative number, the difference is a positive number. A given clinical test may be identified as not having been affected by the failure (and, thus, as the reference clinical test) if the one or more requirements are met.

In a particular example, the reference clinical test may be the (chronologically) last clinical test of the plurality of clinical tests that was not affected by the failure of the laboratory instrument. In other words, the reference clinical test may be the last clinical test performed before the failure occurrence timepoint. Accordingly, each clinical test of the plurality of clinical tests that is chronologically subsequent to the reference clinical test was affected by the failure of the laboratory instrument.

In this particular example, identifying the reference clinical test may comprise identifying a pair of chronologically adjacent clinical tests among the plurality of clinical tests, wherein the first clinical test was not affected by the failure of the laboratory instrument and the second clinical test was affected by the failure of the laboratory instrument. The first clinical test is chronologically antecedent to the second clinical test and "chronologically adjacent clinical tests" means that there is no other clinical test in the set of potentially affected clinical tests that was performed after the first clinical test and before the second clinical test. The first clinical test is the reference clinical test and the second clinical test may be referred to as the "first-after-failure clinical test", since it is the first clinical test that was carried out after the failure occurrence timepoint.

Identifying the pair of chronologically adjacent clinical tests may comprise finding the pair, e.g. by searching among the set of potentially affected clinical tests until such a pair is found. The tests in the pair may be found at different moments in time. Exemplarily, a given clinical test may be identified as having been affected by the failure if the one or more requirements discussed above are not met.

The method further comprises determining a proper subset of the set of potentially affected clinical tests based on the reference clinical test and causing the proper subset to be rerun, i.e. to be performed again. The computing device uses the reference clinical test to select a proper subset of the plurality of clinical tests, wherein the proper subset contains less elements than the set of potentially affected clinical tests. The proper subset comprises the clinical tests of the plurality of clinical tests that are designated to be rerun as a consequence of the failure of the laboratory instrument. Accordingly, each clinical test in the proper subset is performed at least twice, namely at least one more time in addition to the initial run.

As discussed above, the identification of the reference clinical test narrows down the time frame in which the failure occurrence timepoint lies and enables the computing device to exclude some clinical tests of the set of potentially affected clinical tests from the rerun operations, because it is established that at least these tests have not been affected by the failure.

In a particular example, the proper subset of clinical tests may comprise the clinical tests that are chronologically subsequent to the reference clinical test. In case the reference clinical test is the last clinical test performed before the failure occurrence timepoint, the proper subset may comprise only clinical tests that were affected by the failure. Otherwise, the proper subset may comprise both clinical tests that were affected and not affected by the failure.

More particularly, the proper subset of clinical tests may comprise the clinical tests that are chronologically subsequent to the reference clinical test and that have yet to be rerun. Indeed, in some cases the identification of the reference clinical test may comprise rerunning (by a properly functioning laboratory instrument) one or more of the clinical tests in the set of potentially affected clinical tests, as discussed below. These tests that have already been rerun may be excluded from the proper subset, even if they were performed after the reference clinical test. Exemplarily, the proper subset of clinical tests may consist of the clinical tests that are chronologically subsequent to the reference clinical test and that have yet to be rerun.

In another example, the proper subset of clinical tests may comprise the clinical tests that are chronologically subsequent to the reference clinical test together with a predetermined number of clinical tests that are chronologically antecedent to the reference clinical test. Also in this example, tests that have already been rerun by a properly functioning laboratory instrument may be excluded from the subset. The inclusion of the chronologically antecedent clinical tests may be considered to be a buffer that allows for rerunning the clinical tests affected by the failure even if the identification of the reference clinical test was not accurate.

The computing device may cause the (same) laboratory instrument to rerun the tests in the proper subset or another laboratory instrument to rerun the tests in the proper subset. In the latter case, the other laboratory instrument may be part of the same laboratory or of a different laboratory. In the former case, the failure of the laboratory instrument has been addressed in the meantime. Accordingly, the method may comprise, prior to causing the proper subset of clinical tests to be rerun, addressing (e.g. by a user) the failure of the laboratory instrument and determining that the laboratory instrument functions correctly again. In other words, the clinical tests of the proper subset are then performed again by the same laboratory instrument that performed them the first time.

Exemplarily, the flow of the quality control routine may comprise, when a failure of the laboratory instrument is determined, the following steps besides the steps discussed above:

- identifying, by the user, the type of failure (e.g. which component of the laboratory instrument failed);
- investigating, by the user, the cause of the failure based on the type of failure;
- addressing, by the user, the failure based on the cause of the failure (e.g. this may include recalibration of the laboratory instrument, checking and replacing reagents, ensuring proper maintenance of the laboratory instrument);
- determining, by the computing device and/or the user, whether the laboratory instrument functions correctly again.

The step of determining whether the laboratory instrument functions correctly again may comprise:

- making, by the user, a container containing (second) control material available to the laboratory instrument (e.g. loading the container in the laboratory instrument or into an input unit or a transport unit connected to the laboratory instrument);
- causing, by the computing device, the laboratory instrument to perform at least one (second) control test on the (second) control material to obtain (second) control result data;
- evaluating, by the computing device, the (second) control result data against the positive and/or negative criteria.

In some examples, the proper subset may contain at least one clinical test. In other examples, the proper subset may be empty. In this case, the failure may have been determined shortly after it occurred, so that no clinical tests were carried out between the failure determination timepoint and the failure occurrence timepoint. If the proper subset is empty, the step of "causing the proper subset to be rerun" amounts to not causing any clinical test to be rerun.

According to the method of the present invention, the number of clinical tests that are rerun in case of a failure of a laboratory instrument is reduced with respect to the conventional case. Indeed, instead of rerunning all the clinical tests in the set of potentially affected clinical tests, only a proper subset thereof is rerun. This is possible thanks to the identification of the reference clinical test, which leads to the identification of at least some clinical tests that need not be rerun. Reducing the number of clinical tests that are rerun saves the time and resources (including reagents and sample material) that would have been needed to rerun the additional clinical tests, while at the same time ensuring that the test results are correct. Therefore, the sample testing is made more efficient while maintaining the accuracy.

Exemplarily, the method may further comprise rerunning the proper subset of clinical tests consequently to the computing device causing the rerun. As mentioned, the rerunning may be performed by the same laboratory instrument or another laboratory instrument.

In a particular example, identifying the reference clinical test may comprise:

- performing an identification procedure for a check clinical test comprising:

  -- selecting the check clinical test from the plurality of clinical tests;
  -- performing a comparison between a comparison value obtained from one or more test results of the plurality of test results and one or more reference values, wherein the one or more test results of the plurality of test results comprise the test result associated with the check clinical test;
  -- checking whether, based on the comparison, one or more sets of predetermined conditions are satisfied;

- when none of the sets of predetermined conditions is satisfied, repeating, by the computing device, the identification procedure for at least another check clinical test until one of the sets of predetermined conditions is satisfied;
- when one of the sets of the predetermined conditions is satisfied, setting, by the computing device, the reference clinical test based on the check clinical test.

In this particular example, the computing device chooses a clinical test from the set of potentially affected clinical tests as a first or possibly only check clinical test. If the chosen clinical test has certain properties, as codified by the one or more sets of predetermined conditions, then the reference clinical test has been found. Otherwise, the computing device chooses another clinical test from the set of potentially affected clinical tests as a second check clinical test. Again, if the chosen clinical test has certain properties, as codified by the one or more sets of predetermined conditions, then the reference clinical test has been found. Otherwise, the computing device chooses yet another clinical test from the set of potentially affected clinical tests as a third check clinical test. This goes on until the reference clinical test is found.

In short, the method may comprise a loop (the identification procedure), i.e. a sequence of steps that can be potentially repeated multiple times, wherein the exit condition of the loop is given by meeting one of the one or more sets of predetermined conditions. Every time that the identification procedure is repeated, a different clinical test is selected from the set of potentially affected clinical tests as check clinical test. In particular, the identification procedure is never performed twice for the same clinical test and the check clinical test varies over time, namely from identification procedure to identification procedure.

The first step of the identification procedure is selecting a given clinical test, which is referred to as the check clinical test, from the plurality of clinical tests. The choice of the check clinical test among all possible clinical tests may be made according to a search algorithm, wherein the search algorithm defines which clinical test should be chosen first as check clinical test and, if the identification procedure has to be repeated, to which clinical test to move next as the check clinical test. In other words, if the set of potentially affected clinical tests comprises $N$ tests and the check clinical test is denoted with $T_c$, the search algorithm determines how to choose c among the possible values 1 to $N$ every time that the identification procedure is performed. Exemplarily, the selection of the check clinical test may depend on the outcome of one or more of the previously performed identification procedures. Some examples for a search algorithm are discussed below.

The second step of the identification procedure is performing a comparison between a comparison value obtained from one or more test results of the plurality of test results and one or more reference values, wherein the one or more test results of the plurality of test results comprise the test result associated with the check clinical test. Accordingly, the comparison value is derived at least from the test result associated with the check clinical test currently under consideration. Thus, each check clinical test is associated with a respective comparison value, including the check clinical test that will turn out to be the reference clinical test. That is why, as exemplarily described above, identifying the reference clinical test may comprise at least performing a comparison between the comparison value associated with the reference clinical test and the one or more reference values.

If the first check clinical test is indeed the reference clinical test, no other comparisons may be performed. If not, a plurality of comparisons may be performed, one for each identification procedure, each of the comparisons involving a different comparison value, since the comparison value depends on the test result associated with the specific check clinical test. In some examples, the one or more reference values may be the same for all comparisons, while in other examples each comparison value may be compared to respective one or more reference values, which may be different for each comparison value.

As described above, performing a comparison may comprise computing a difference between the comparison value and (each of) the reference value(s).

In a particular example, the comparison value for a given check clinical test may be an exponentially weighted moving average and the exponentially weighted moving average may be obtained from a subgroup of the plurality of test results including the test results associated with the check clinical test and with clinical tests chronologically preceding it. More generally, the EWMA may be obtained from a group of test results including the test result associated with the check clinical test and test results associated with clinical tests chronologically preceding it, wherein this group of test results may also include test results associated with clinical test carried out by the laboratory instrument but not in the set of potentially affected clinical tests, e.g. clinical tests carried out prior to the last timepoint at which it was determined that the laboratory instrument was functioning correctly. The number of clinical tests chronologically preceding the check clinical test whose result are included in the EWMA as well as other parameters needed for computing the EWMA may be set by a user or by the computing device, e.g. on the basis of machine learning. Exemplarily, all clinical tests (e.g. all tests performed by that laboratory instrument since it started operating or all tests performed by that laboratory instrument from a refresh point at which the previous clinical tests are deleted) preceding the check clinical test are included in the EWMA.

When the comparison value is the EWMA, the one or more reference values may be the same for all comparison values, i.e. for all check clinical tests. For instance, the one or more reference values may be an upper limit, *U*, and a lower limit, *L*, the upper limit and lower limit being derived from a standard deviation, S, and an average, A, of a set of benchmark test results for the clinical test. In particular, the following relations hold:

$$U = A + \alpha\, S, \qquad L = A - \alpha\, S,$$

wherein a = 2 or 3.

In another particular example, the comparison value for a given check clinical test may be the test result associated with the check clinical test itself. In this case, the identification procedure for the check clinical test may further comprise, after selecting the check clinical test and prior to performing the comparison, causing the check clinical test to be rerun to obtain a rerun test result. Similarly to what discussed above, the check clinical test may be rerun by the same laboratory instrument, if it has been fixed, or by a different laboratory instrument.

In this example, the one or more reference values may be obtained from the rerun test result. Accordingly, in this case, the reference values may be different for each comparison value. In particular, the one or more reference values may be an upper limit and a lower limit defining an interval including the rerun test result. For instance, the upper limit and the lower limit may be obtained by adding to and subtracting from, respectively, the rerun test result a value accounting for measurement uncertainty.

The identification procedure further comprises checking whether, based on the comparison, one or more sets of predetermined conditions are satisfied. In other words, it is checked whether the relation between the comparison value and the reference value(s) meets certain predetermined condition(s). Exemplarily, checking whether the one or more sets of predetermined conditions are satisfied may comprise determining whether the check clinical test was affected by the failure or not.

In particular, a predetermined condition may comprise a mathematical or a logical expression to be evaluated by using the comparison value and the reference value(s). If this expression is evaluated to be true, the condition may be considered satisfied.

A set of predetermined conditions may comprise one or more predetermined conditions, and there may be one or more sets that are checked. A set of predetermined conditions is considered satisfied when each condition within the set is satisfied. If it cannot be determined whether a condition is satisfied, e.g. because of a lack of information at the moment in which the check is performed, the condition is considered not satisfied. Said otherwise, an indeterminable condition is deemed not satisfied. This may happen in cases in which the conditions refer to different clinical tests and one of these has not yet been selected as check clinical test, as discussed below.

When no set of predetermined conditions of the one or more sets is satisfied, the identification procedure is repeated for one or more other check clinical tests until one set of predetermined conditions is satisfied. When one set of predetermined conditions of the one or more sets is satisfied, the reference clinical test is set based on the check clinical test. In some examples, the reference clinical test may be the check clinical test of the identification procedure performed last, in other examples the reference clinical test may be the clinical test immediately preceding the check clinical test of the identification procedure performed last.

In a particular example, the one or more sets of predetermined conditions may comprise one single set including a first condition that the check clinical test was not affected by the failure of the laboratory instrument. In some instances, the one or more predetermined conditions may consist of the first condition.

In this example, the computing device searches through the plurality of clinical tests until it finds one that was not affected by the failure of the laboratory instrument, i.e. that was performed before the failure occurrence timepoint. For instance, if the comparison value is the test result itself, a clinical test may be considered unaffected if the test result is consistent with the rerun test result (e.g. within a confidence interval defined by the reference values), and affected if the test result is not consistent with the rerun test result. If the comparison value is the EWMA, a clinical test may be considered unaffected if the EWMA behaves as expected (e.g. remains within a range defined by the reference values) and affected if the EWMA does not behave as expected. It should be noted that the states of being affected and unaffected are mutually exclusive and there is no other third state.

By identifying the reference clinical test according to this particular example, the identification may be particularly time-efficient. For instance, in a best-case scenario, the reference clinical test may be identified by performing only one identification procedure.

In another particular example, the one or more sets of predetermined conditions may comprise a first set of predetermined conditions and a second set of predetermined conditions. The first set of predetermined conditions may comprise a first condition that the check clinical test was not affected by the failure of the laboratory instrument, and a second condition that a clinical test immediately chronologically following the check clinical test was affected by the failure of the laboratory instrument. The second set may comprise a third condition that the check clinical test was affected by the failure of the laboratory instrument, and a fourth condition that a clinical test immediately chronologically preceding the check clinical test was not affected by the failure of the laboratory instrument.

The first set and the second set may be used equivalently to identify as the reference clinical test the last clinical test performed before the failure occurrence timepoint. In this example, there are two conditions on two different clinical tests that are chronologically adjacent, wherein one is the reference clinical test and the other one is the first-after-failure clinical test, as discussed above. Accordingly, in this example the computing device carries out at least two identification procedures.

For example, in some cases the identification procedure is performed an *n*-th time (with n≥1), e.g., for an *n*-th check clinical test, and allows for concluding that the *n*-th check clinical test has not been affected by the failure. Moreover, the identification procedure is performed an *m*-th time (with m≥2), e.g., for an *m*-th check clinical test, wherein the *m*-th performance of the identification procedure is carried out after the *n*-th performance identification procedure and the *m*-th check clinical test immediately follows the *n*-th check clinical test in the chronological sequence of the plurality of clinical tests. If the identification procedure for the *m*-th check clinical test allows for concluding that the *m*-th check clinical test has been affected by the failure, then the first set of conditions is satisfied, and the *n*-th check clinical test and the *m*-th check clinical test are respectively set to the reference clinical test and the first-after-failure clinical test.

For example, in other cases the identification procedure is performed an *n*-th time (with n≥1), e.g., for an *n*-th check clinical test, and allows for concluding that the *n*-th check clinical test has been affected by the failure. Moreover, the identification procedure is performed an *m*-th time (with m≥2), e.g., for an *m*-th check clinical test, wherein the *m*-th performance of the identification procedure is carried out after the *n*-th performance identification procedure and the *m*-th check clinical test immediately precedes the *n*-th check clinical test in the chronological sequence of the plurality of clinical tests. If the identification procedure for the *m*-th check clinical test allows for concluding that the *m*-th check clinical test has not been affected by the failure, then the second set of conditions is satisfied, and the *n*-th check clinical test and the *m*-th check clinical test are respectively set to the first-after-failure clinical test and the reference clinical test.

By identifying the reference clinical test according to this particular example, the rerun may be particularly time- and resource-efficient, since the proper subset is minimised.

In particular, when the one or more reference values comprise an upper limit and a lower limit, it may be determined that a given clinical test was not affected by the failure of the laboratory instrument when the respective comparison value is within a numerical range comprised between the lower limit and the upper limit, and that the given clinical test was affected by the failure of the laboratory instrument when the respective comparison value is outside a numerical range comprised between the lower limit and the upper limit. The numerical range comprised between the lower limit and the upper limit may or may not include the lower limit and/or the upper limit, i.e. it may be an open or a (semi-)closed range.

Accordingly, a condition may be mathematically expressed as an inequality. With regards to the exemplary conditions discussed above, it may be said that the first condition requires that the comparison value of the check clinical test be within a numerical range comprised between the lower limit and the upper limit. Similarly, the second condition may require that the comparison value of the clinical test immediately chronologically following the check clinical test be outside of a numerical range comprised between the lower limit and the upper limit. As explained before, the reference value(s) may vary from check clinical test to check clinical test, so that the lower and upper limits may be different in the first and second conditions.

The same applies analogously for the third and fourth conditions.

In some particular examples, double-check measures may be implemented in order to ensure that the clinical test identified as reference clinical test is indeed the reference clinical test, i.e. satisfies the properties of the reference clinical test.

For example, in the cases discussed above in which the comparison value is the test result associated with the check clinical test, and the one or more reference values are obtained from the rerun test result, the method may further comprise causing the check clinical test to be rerun a second time to obtain a second rerun test result and the comparison value may be compared also with one or more second reference values obtained from the second rerun test result.

In particular, in this case, the steps of causing the check clinical test to be rerun, performing the comparison and checking whether the one or more sets of predetermined conditions are satisfied may be performed twice. Accordingly, in these cases, identifying the reference clinical test may comprise:

- performing an identification procedure for a check clinical test comprising:

  -- selecting the check clinical test from the plurality of clinical tests;
  -- causing the check clinical test to be rerun to obtain a (first) rerun test result;
  -- performing a (first) comparison between the test result associated with the check clinical test and one or more (first) reference values obtained from the (first) rerun test result;
  -- checking whether, based on the (first) comparison, one or more sets of predetermined conditions are satisfied;
  -- causing the check clinical test to be rerun a second time to obtain a second rerun test result;
  -- performing a second comparison between the test result associated with the check clinical test and one or more second reference values obtained from the second rerun test result;
  -- checking whether, based on the second comparison, the one or more sets of predetermined conditions are satisfied;

  if checking whether the one or more sets of predetermined conditions are satisfied based on the (first) comparison and the second comparison yields the same result:

- when none of the sets of the predetermined conditions is satisfied, repeating, by the computing device, the identification procedure for at least another check clinical test until each of the one or more predetermined conditions is satisfied;
- when one of the sets of the predetermined conditions is satisfied, setting, by the computing device, the reference clinical test based on the check clinical test.

For instance, if checking whether the one or more sets of predetermined conditions are satisfied based on the (first) comparison and the second comparison does not yield the same result, a third rerun of the check clinical test may be performed to resolve the discrepancy between the check based on the first comparison and the check based on the second comparison.

In a more particular example, identifying the reference clinical test may comprise:

- performing an identification procedure for a check clinical test comprising:

  -- selecting the check clinical test from the plurality of clinical tests;
  -- causing the check clinical test to be rerun to obtain a (first) rerun test result;
  -- performing a (first) comparison between the test result associated with the check clinical test and one or more (first) reference values obtained from the (first) rerun test result;
  -- checking whether, based on the (first) comparison, one or more sets of predetermined conditions are satisfied;

if, based on the checking, it was determined that the check clinical test was affected by the failure of the laboratory instrument:

--- causing the check clinical test to be rerun a second time to obtain a second rerun test result;
--- performing a second comparison between the test result associated with the check clinical test and one or more second reference values obtained from the second rerun test result;
--- checking whether, based on the second comparison, the one or more sets of predetermined conditions are satisfied;

if checking whether the one or more sets of predetermined conditions are satisfied based on the (first) comparison and the second comparison yields the same result, or if no second comparison was performed:

- when none of the sets of the predetermined conditions is satisfied, repeating, by the computing device, the identification procedure for at least another check clinical test until each of the one or more predetermined conditions is satisfied;
- when one of the sets of the predetermined conditions is satisfied, setting, by the computing device, the reference clinical test based on the check clinical test.

Performing a second rerun may avoid "false positive/negative" results due to potential noise in the testing carried out by laboratory instruments.

In other examples, additional conditions may be included in a set. For instance, the single set discussed above may include the first condition that the check clinical test was not affected by the failure of the laboratory instrument and, additionally, the fourth condition that a clinical test immediately chronologically preceding the check clinical test was not affected by the failure of the laboratory instrument.

In another instance, the first set discussed above may comprise the first condition that the check clinical test was not affected by the failure of the laboratory instrument, the second condition that a clinical test immediately chronologically following the check clinical test was affected by the failure of the laboratory instrument and a fifth condition that a clinical test immediately chronologically following the clinical test immediately chronologically following the check clinical test was affected by the failure of the laboratory instrument.

The second set may comprise the third condition that the check clinical test was affected by the failure of the laboratory instrument, the fourth condition that a clinical test immediately chronologically preceding the check clinical test was not affected by the failure of the laboratory instrument, and the second condition that a clinical test immediately chronologically following the check clinical test was affected by the failure of the laboratory instrument.

In a particular example, identifying the reference clinical test comprises using a dichotomic search algorithm based on the one or more sets of predetermined conditions. Thus, the selection of the check clinical test follows a dichotomic search algorithm.

Considering the set of potentially affected clinical tests $\{T_1, ..., T_N\}$, the check clinical test selected for the first (and potentially only) iteration of the identification procedure may be $T_i$ with $$i = integer \left[\frac{N+1}{2}\right]$$, wherein the *integer* function may be the floor function or the ceiling function. If one set of predetermined conditions is satisfied for this check clinical test $T_i$, then $T_i$ is the reference clinical test.

If not, the iteration procedure is repeated for another check clinical test, which may be selected as $T_j$, wherein $$j = integer \left[\frac{i+1}{2}\right]$$ if $T_i$ was determined to be affected by the failure of the laboratory instrument and $$j = integer \left[\frac{i+N}{2}\right]$$ if $T_i$ was determined not to be affected by the failure of the laboratory instrument. If one set of predetermined conditions is satisfied for this check clinical test $T_j$, the reference clinical test can be identified based on $T_j$.

If not, the iteration procedure is repeated for yet another check clinical test which may be selected as $T_k$, wherein:

- $$k = integer \left[\frac{j+1}{2}\right]$$ if $T_j$ was determined to be affected by the failure of the laboratory instrument and $j < i$;

- $$k = integer \left[\frac{j+i}{2}\right]$$ if $T_j$ was determined to be affected by the failure of the laboratory instrument and $j > i$;

$$k = integer \left[\frac{j+N}{2}\right]$$ if $T_j$ was determined not to be affected by the failure of the laboratory instrument and $j > i$;

$$k = integer \left[\frac{j+i}{2}\right]$$ if $T_j$ was determined not to be affected by the failure of the laboratory instrument and $j < i$.

If one set of predetermined conditions is satisfied for this check clinical test $T_k$, the reference clinical test can be identified based on $T_k$. Otherwise, the search keeps going with $T_h$, wherein $h$ can be chosen based on $k$ in the same way $k$ was chosen based on $j$, and so on.

More generally, the dichotomic search algorithm may be illustrated by referring not only to the set of potentially affected clinical tests $\{T_1, ..., T_N\}$, but also to the set of check clinical tests. The set of check clinical tests is the set that contains each clinical test of the set of potentially affected clinical tests that has been at one point selected to be a check clinical test. Before the first identification procedure, the set is empty and then one new element is added every time that the identification procedure is performed. Thus, the number of elements of the set of check clinical tests is equal to the number of iterations of the identification procedure.

Exemplarily, instead of using the dichotomic search algorithm over the whole set of potentially affected clinical tests, the method may comprise first performing the identification procedure for the last test in the chronological sequence, $T_N$. In this way, if $T_N$ is the reference clinical test, it may be determined that none of the clinical tests were affected by the failure. In other words, the proper subset may be empty, as discussed above. If $T_N$ is not the reference clinical test, the dichotomic search algorithm may then be applied to the subset of clinical tests $\{T_1, ..., T_{N-1}\}$.

The check clinical tests may be denoted as $\{C^0, ..., C^P\}$, wherein $\forall a = 0, ..., P \exists b = 1, ..., N$ s. t. $C^a = T_b$ and $P < N$. A function $index$ may be defined, wherein $b = index(C^a)$. Two consecutive numbers denote two check clinical tests that were chosen immediately consecutive to one another. Accordingly, $C^0$ is the clinical test of the plurality of clinical tests that is first selected as check clinical test, $C^1$ is the clinical test of the plurality of clinical tests that is selected as check clinical test right after $C^0$, i.e., the identification procedure for $C^1$ is performed immediately after the identification procedure for $C^0$, and so on.

Exemplarily, as discussed above, it is possible to set $C^0 = T_N$, and $C^1 = T_i$ with $i = integer \left[\frac{N}{2}\right]$. Hence, in particular, in this case, $N = index(C^0)$ and $integer \left[\frac{N}{2}\right] = index(C^1)$. If $C^1$ is not the reference clinical test, one or more additional repetitions of the identification procedure are performed. The function $index$ for $a > 1$ reads as follows:

$$index(C^a) = integer \left[\frac{index(C^{a-1})+x}{2}\right].$$

In particular, if $C^{a-1}$ was affected by the failure of the laboratory instrument the number x may be equal to either:

- $index(C^z) + 1$ if there is a $C^z$, with $0 \leq z < a$, in the set of check clinical tests such that $index(C^z) < index(C^{a-1})$, and in case there is a plurality of check clinical tests $C^{z1}, C^{z2}, ...$ satisfying this condition, then x is equal to the max$[index(C^{z1}), index(C^{z2}), ...] + 1$; or
- 1 otherwise.

Moreover, in particular, if $C^{a-1}$ was not affected by the failure of the laboratory instrument, the number x may be equal to either:

- $index(C^z) - 1$ if there is a $C^z$, with $0 \leq z < a$, in the set of check clinical tests such that $index(C^z) > index(C^{a-1})$, and in case there is a plurality of check clinical tests $C^{z1}, C^{z2}, ...$ satisfying this condition, then x is equal to the min $[index(C^{z1}), index(C^{z2}), ...] - 1$; or
- $N$ otherwise.

In other examples, the search algorithm may be a linear search algorithm or a jump algorithm. The use of the dichotomic search algorithm makes the identification of the reference clinical test efficient, thereby increasing the time efficiency of the whole method,

As discussed above, accessing the test data may comprise selecting the plurality of clinical tests based on one or more constraints. In a particular example, a constraint may be a constraint on each test result of the plurality of test results. This may be referred to as the "result constraint". Exemplarily, the one or more constraints may comprise the result constraint and the time range constraint. In this case, the set of potentially affected clinical tests may comprise those clinical tests within the time range and whose test results satisfy the result constraint.

For instance, the result constraint may require that the test result of a clinical test be within a prescribed range (open range or close range). It may indeed happen that the failure of the laboratory instrument only affects results in a given range, corresponding e.g. to a given concentration of an analyte. In this case, any clinical test that was performed before the failure occurrence timepoint was not affected by the failure, while any clinical test that was performed after the failure occurrence timepoint and whose test result is in the given range was affected by the failure of the laboratory instrument. Clinical tests performed after the failure occurrence timepoint and whose test results are not in the given range were not affected by the failure of the laboratory instrument. Exemplarily, the spectrum of test results for a clinical test may be divided into a low range, a mid range and a high range.

In a further particular example, a constraint may be a constraint on a moving average (e.g. the EWMA) of a set of test results. This may be referred to as the "average constraint". In particular, the average constraint may require that a clinical test have the moving average computed up to its test result lying outside a predefined range, e.g., the range comprised between the upper limit, $U$, and the lower limit, $L$, defined above.

One or more clinical tests satisfying the average constraint may be identified and, among these, the temporally first (i.e. the one performed first in time) clinical test may be identified. The latter clinical test may be denoted as outlier clinical test. If the clinical tests are proofed according to their chronological order, the outlier clinical test is the only one identified as satisfying the average constraint. In this case, the set of potentially affected clinical tests may comprise (or consist of) all clinical tests within the time range that are chronologically subsequent to the outlier clinical test, the outlier clinical test itself, and, optionally, a predetermined number (e.g. 10, or 5, or 3) of clinical tests within the time range that are chronologically antecedent to the outlier clinical test. The predetermined number of clinical tests is such that it is a proper subset of all chronologically antecedent clinical tests within the time range.

Exemplarily, the one or more constraints may comprise the average constraint and the time range constraint. In this case, the comparison value discussed above may be the test result.

Accordingly, in this example selecting the plurality of clinical tests based on the average constraint, the average constraint requiring that a clinical test have the moving average computed up to its test result lying outside a predefined range, may comprise:

- identifying an outlier clinical test, wherein the outlier clinical test satisfies the average constraint and there is no clinical test preceding the outlier clinical test that satisfies the average constraint;
- including in the plurality of clinical tests the outlier clinical test, all clinical tests that are chronologically subsequent to the outlier clinical test and, optionally, a predetermined number of clinical tests that are chronologically antecedent to the outlier clinical test.

In another particular example, a constraint may be a constraint on the type of test. For instance, the failure may be due to the contamination of a reagent that is only used in a particular type of test. Accordingly, the set of potentially affected clinical tests may comprise those clinical tests within the time range and whose type meets the constraint, e.g. whose type is the type set in the constraint.

In yet another example, a constraint may be a constraint on the type of sample container containing the biological sample on which the clinical tests is carried out. For instance, a mechanical malfunction of the laboratory instrument may cause an incorrect handling of only certain types of sample containers, e.g. having certain dimensions. Accordingly, the set of potentially affected clinical tests may comprise those clinical tests within the time range and whose type of sample container meets the constraint, e.g. whose type is the type set in the constraint.

All the exemplary constraints discussed herein may be optionally considered in any combination.

The one or more constraints may be determined based on the failure of the laboratory instrument, e.g. based on the cause of the failure or the type of failure. The constraint(s) may be set by a user and/or by the computing device.

Using one or more constraints to select the set of potentially affected clinical tests increases the time efficiency of the method both at the level of identifying the reference clinical test and at the level of rerunning the subset of clinical tests.

A second aspect of the present invention relates to a computing device comprising a processor configured to perform the method described herein.

A third aspect of the present invention relates to an automated laboratory system comprising the computing device according to the second aspect. Exemplarily, the automated laboratory system may comprise the laboratory instrument described herein when discussing the first aspect of the present invention. The laboratory instrument may comprise the computing device according to the previous aspect or the computing device may be external to the laboratory instrument.

According to a fourth aspect of the invention, a computer program product is provided. The computer program product comprises instructions which, when the program is executed by a computer, cause the computer to carry out the method described herein. In particular, the computer may be the computing device according to the second aspect of the present invention.

A fifth aspect of the present invention refers to a computer-readable medium, e.g. a transitory computer readable medium comprising instructions which, when executed by a computer, cause the computer to carry out the method described herein. In particular, the computer may be the computing device according to the second aspect of the present invention.

Brief Description of the Drawings

Details of exemplary embodiments are set forth below with reference to the exemplary drawings. Other features will be apparent from the description, the drawings, and from the claims. It should be understood, however, that even though embodiments are separately described, single features of different embodiments may be combined to further embodiments.

Figure 1 shows a schematic representation of a laboratory instrument and a computing device.

Figure 2 shows a flow chart of an exemplary method.

Figure 3 shows a flow chart of an exemplary identification of a reference clinical test in the method of figure 2.

Figures 4(a) to 4(e) show a visual representation of an exemplary dichotomic search.

Detailed Description

In the following, a detailed description of examples will be given with reference to the drawings. It should be understood that various modifications to the examples may be made. Unless explicitly indicated otherwise, elements of one example may be combined and used in other examples to form new examples.

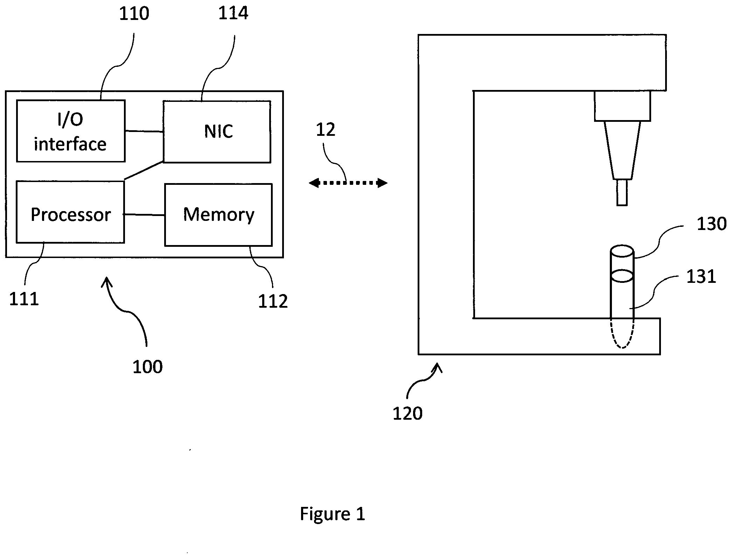

**Figure 1** shows a schematic representation of an laboratory instrument 120 and a computing device 100. In one example, the laboratory instrument 120 comprises the computing device 100. In another example, the computing device 100 is separate from the laboratory instrument 120. In particular, the computing device 100 is configured to control the laboratory instrument 120, which is configured to analyse biological samples.

Exemplarily, the computing device 100 comprises a processor 111 (e.g., a CPU, a GPU, or the like), and a memory 112. The processor 111 is configured to carry out the method described herein. The memory 112 may comprise a primary memory and a secondary memory (not shown). In particular, the secondary memory stores a computer program comprising instructions which, when executed by the processor 111, cause the computing device 100 to carry out the method described herein. The computing device 100 may also comprise a input output (I/O) interface 110 for communicating with input/output units, such as a screen, a keyboard, a touch screen, a printer, or the like.

Furthermore, the computing device 100 may comprise a Network Interface Controller (NIC) 114 configured to connect said device with one or more networks (e.g. an intranet, the internet, a cellular network, or the like). In other examples, the computing device 110 may comprise a plurality of NICs.

The computing device 100 is in data communication 12 with the laboratory instrument 120, for example by means of the NIC 114 or by other means when the computing device 100 is separate from the laboratory instrument 120. Alternatively, the computing device 100 may be a part of the laboratory instrument 120 and the data transfer may occur via a bus or an internal network. The computing device 100 is configured to carry out the method according to the present disclosure.

The laboratory instrument 120 is configured to carry out one or more clinical tests on biological samples. In particular, a biological sample 131 may be contained in a sample container 130, such as a sample tube, and the laboratory instrument 120 comprises means to receive and handle the sample container 130.

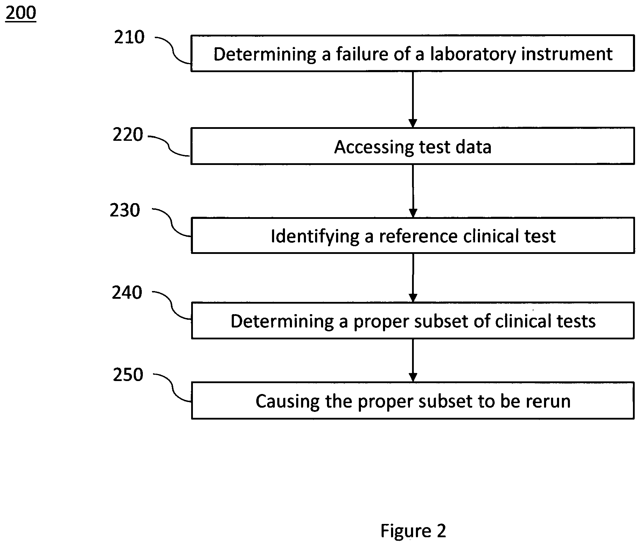

In particular, the computing device 100 is configured to carry out the method 200 illustrated in **Figure 2.**

The method 200 comprises, at 210, determining a failure of the laboratory instrument 120. In particular, the computing device 100 can ascertain that the failure of the laboratory instrument 120 has occurred in the context of a quality control routine, which is performed at regular intervals, e.g. every s clinical tests, wherein, typically, the number s may be set by a user of the laboratory instrument e.g., based on the laboratory instrument and the stability of this laboratory instrument in carrying out the clinical tests. The computing device 100 can inform a user when s clinical tests have been performed since the last quality control routine and the user may arrange for a control material to be analysed by the laboratory instrument 120. If the analysis of the control material does not return an expected result associated with the control material, the computing device 100 determines the failure of the laboratory instrument 120.

The method 200 further comprises, at 220, accessing test data comprising information specifying a plurality of clinical tests that were carried out by the laboratory instrument 120 and that may have been affected by the failure of the laboratory instrument 120, a chronological sequence of the plurality of clinical tests, and a respective plurality of test results associated with the plurality of clinical tests.

The information specifying the plurality of clinical tests may comprise respective test identifiers, each test identifier being associated with and uniquely identifying a respective clinical test. The test identifiers may be alphanumeric strings. Furthermore, for each clinical test in the set of potentially affected clinical tests, the information may comprise sample information identifying the sample on which the respective clinical test was conducted, e.g. a sample identifier such as an alphanumeric string. The information specifying the plurality of test results may comprise a plurality of numerical values. The clinical tests may be arranged in a chronologically ordered list that provides the information about the chronological sequence.

The plurality of clinical tests lies within a time range having a start timepoint and an end timepoint, wherein, in particular, the end timepoint is the access timepoint. For instance, the start timepoint is the moment in time at which it was last determined that the laboratory instrument was functioning correctly (e.g. the moment at which the previous quality control routine with positive outcome was completed). Accordingly, the failure occurrence timepoint must be between the start timepoint and the end timepoint.

In some examples, the quality control routine may be a so-called multi-level quality control routine, which involves using different levels of the control materials that mimic patient samples. At different levels, such as high, low, and medium, control materials are prepared to cover a range of analyte concentrations, so as to assess the performance of the laboratory instrument 120 at the respective level. The high-level control material contains analyte concentrations at the upper end of the normal range or above. The low-level control material, on the other hand, contains analyte concentrations at the lower end of the normal range or below. The medium-level control material falls within the normal range of analyte concentrations.

If it is determined that the failure of the laboratory instrument 120 impacts only test results in one range, the computing device 100 may choose for the set of potentially affected clinical tests the plurality of clinical tests that lie within the time range and have test results within the affected range.

The method 200 further comprises, at 230, identifying a reference clinical test among the plurality of clinical tests, the reference clinical test being a clinical test that has not been affected by the failure of the laboratory instrument 120. Thus, the computing device 100 searches for and finds a clinical test in the set of potentially affected clinical tests that was not affected by the failure and, thus, was performed before the failure occurrence timepoint.

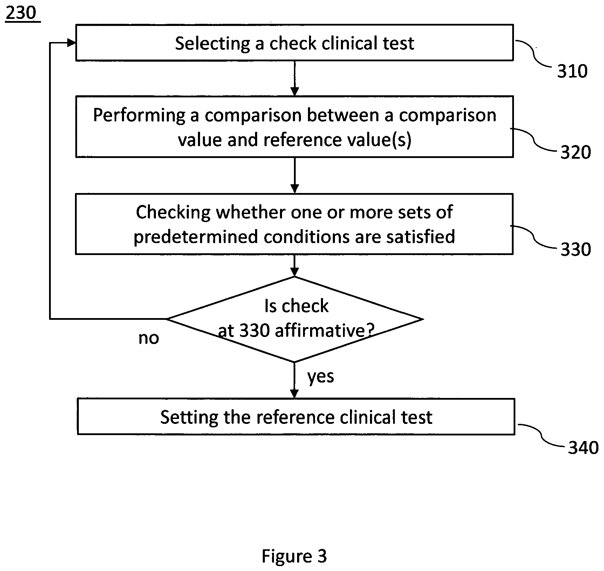

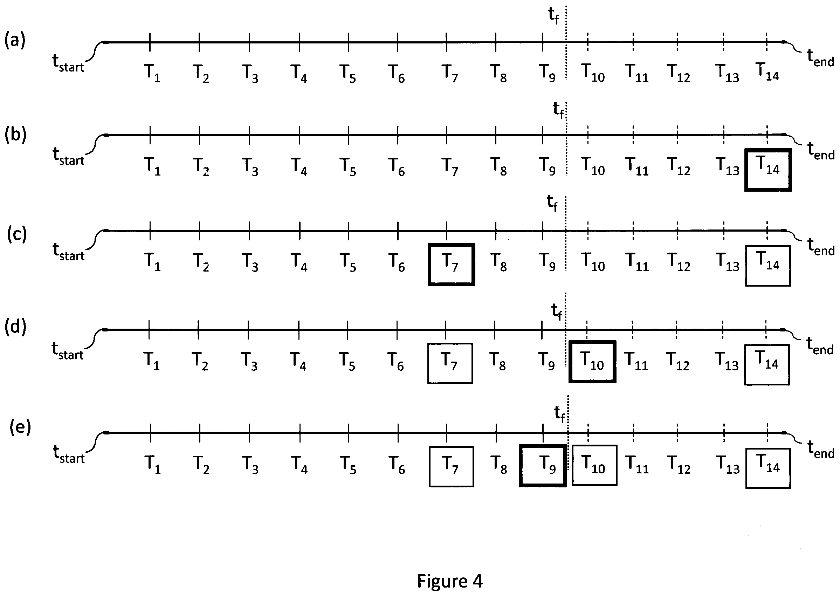

**Figure 3** shows a flow chart of an exemplary identification of the reference clinical test in step 230. In particular, the identification may be done using a dichotomic search algorithm. **Figure 4** shows a visual representation of an exemplary dichotomic search. The example shown in figure 4 is for a set of potentially affected clinical tests $\{T_1, ..., T_N\}$ with $N=14$. The number of tests is purely illustrative and is in no way limiting. The clinical tests are represented in figure 4 by vertical lines on a timeline, wherein the timeline starts at the start timepoint $t_{start}$ and ends at the end timepoint $t_{end}$. Adjacent segments indicate clinical tests that were carried out immediately one after the other. In particular, clinical test $T_i$ was carried out immediately before clinical test $T_{i+1}$, for each $i = 1, ..., N-1$. In particular, clinical test $T_i$ was carried out immediately after clinical test $T_{i-1}$, for each $i = 2, ..., N$. The size of the segments on the time axis is not to scale.

Figure 4(a) shows the ordered sequence of clinical tests, wherein the dotted line indicates the failure occurrence timepoint $t_f$. The clinical tests performed before $t_f$ were not affected by the failure and are represented by solid lines, the clinical tests performed after $t_f$ were affected by the failure and are represented by dashed lines. The computing device 100 searches for a clinical test that was not affected by the failure, in some particular cases for the last clinical test that was not affected.

Identifying the reference clinical test comprises, at 310, selecting a check clinical test from the set of potentially affected clinical tests. In particular, step 310 may be performed a plurality of times. Initially, a first check clinical test $C^0$ may be selected. Figure 4(b) shows that $C^0 = T_{14}$ (current check clinical test is shown as encased in a thick square), i.e. the last clinical test in the chronological sequence.

At 320, the computing device 100 performs a comparison between a comparison value obtained from one or more test results of the plurality of test results and one or more reference values, wherein the one or more test results of the plurality of test results comprise the test result associated with the check clinical test.

Exemplarily, the comparison value may be the test result of test $T_{14}$, $r(T_{14})$. In this example, the computing device 100 causes the check clinical test to be rerun by a correctly functioning laboratory instrument (the same laboratory instrument, after maintenance, or a different one) to obtain a rerun test result, $rer(T_{14})$. The reference values may be $rer(T_{14}) \pm h_{14}$, with $h_{14}$ being predetermined by a user and/or the computing device 100. For instance, for each $i=1, 2, ...,14$, one of the following relations hold: $h_i = 0.01\, rer(T_i)$, $h_i = 0.05\, rer(T_i)$. In particular, for each $i=1, 2,...,14$, one of the following relations hold: $h_i = 2\, S$, $h_i = 3\, S$, wherein $S$ is the standard deviation of a set of benchmark test results for the clinical test.

At 330, the computing device 100 checks whether, based on the comparison, one or more sets of predetermined conditions are satisfied. In one example (hereinafter referred to as example A), there may be only one predetermined condition (i.e. one set with only one element), namely that the check clinical test was not affected by the failure. In another example (hereinafter referred to as example B), there may be two sets of predetermined conditions. The first set comprises a first condition that the check clinical test was not affected by the failure of the laboratory instrument, and a second condition that a clinical test immediately chronologically following the check clinical test was affected by the failure of the laboratory instrument. The second set comprises a third condition that the check clinical test was affected by the failure of the laboratory instrument, and a fourth condition that a clinical test immediately chronologically preceding the check clinical test was not affected by the failure of the laboratory instrument.

The condition that a clinical test was not affected is expressed as the condition that the respective test result is within a predetermined range around the rerun test result. The condition that a clinical test was affected is expressed as the condition that the respective test result is outside the predetermined range around the rerun test result.

Thus, in the specific case, it is e.g. checked whether $rer(T_{14}) - h_{14} < r(T_{14}) < rer(T_{14}) + h_{14}$. In other cases, the interval may be closed or semiclosed. Both for examples A and B, no set of predetermined conditions is satisfied, because $T_{14}$ was affected by the failure (indeed it was performed after tf). Thus, the method goes back to step 310, at which a second check clinical test $C^1$ is selected.

After the first iteration of the identification procedure shown in figure 4(b), the set of check clinical tests is $\{C^0\} = \{T_{14}\}$. Clinical tests that have been chosen in the past as check clinical tests are shown as encased in a thin square.

At the second iteration of the identification procedure, shown in Figure 4(c), the second check clinical test C¹ is

$$T_{\frac{(N-1)+1}{2}} = T_7$$

selected as at 310. Then the computing device 2 100 compares at 320 $r(T_7)$ with $rer(T_7) \pm h_7$ and checks at 330 whether $rer(T_7) - h_7 < r(T_7) < rer(T_7) + h_7$. For example A, the (only) predetermined condition is satisfied, because $T_7$ was not affected by the failure (indeed it was performed before $t_f$). Accordingly, in example A, the dichotomic search stops at figure 4(c) and the computing device 100 goes to step 340, where it sets the current check clinical test, i.e. $T_7$, as the reference clinical test.

For example B, none of the sets of predetermined condition is satisfied. In the first set, only the first condition met, but the second condition is not met because it is not known whether $T_8$ was affected by the failure of the laboratory instrument 120 or not. In the second set, neither the third condition nor the fourth condition are met, the third condition being false and the fourth condition being undeterminable.

Thus, in example B, the method goes back once again to step 310. After the first and second iterations of the identification procedure shown in figures 4(b) and 4(c), the set of check clinical tests is $\{C^0, C^1\} = \{T_{14}, T_7\}$. At the third iteration of the identification procedure, shown in Figure 4(d), the third check clinical test $C^2$ is selected as $T_{10}$ at 310. The

number 10 is given by the index function, i.e. as $index(C^2) = integer\left[\frac{index(C^1)+x}{2}\right] = integer\left[\frac{7+13}{2}\right]$, wherein the integer function is the floor function. Indeed, since $C^1 = T_7$ was not affected by the failure and since there is a clinical test in the set of check clinical tests, $C^0$, such that $index(C^0) > index(C^1)$ because 14 is greater than 7, the value of $x$ is given by $index(C^0) - 1$.

Then the computing device 100 compares at 320 $r(T_{10})$ with $rer(T_{10}) \pm h_{10}$ and checks at 330 whether $rer(T_{10}) - h_{10} < r(T_{10}) < rer(T_{10}) + h_{10}$. Also in this iteration, none of the sets of predetermined condition is satisfied. In the first set, neither the first condition nor the second condition are met, the first condition being false and the second condition being undeterminable. In the second set, only the third condition is met, because $T_{10}$ was affected by the failure (indeed it was performed after $t_f$), but the fourth condition is undeterminable.

Therefore, the method goes back once again to step 310. After the first, second and third iterations of the identification procedure shown in figures 4(b), 4(c) and 4(d), the set of check clinical tests is $\{C^0, C^1, C^2\} = \{T_{14}, T_7, T_{10}\}$. At the fourth iteration of the identification procedure, shown in Figure 4(e), the fourth check clinical test $C^3$ is selected as $T_9$ at

310. The number 9 is given by the index function, i.e. as $index(C^3) = integer\left[\frac{index(C^2)+x}{2}\right] = integer\left[\frac{10+8}{2}\right]$. Indeed, since $C^3 = T_{10}$ was affected by the failure and since there is a clinical test in the set of check clinical tests, $C^1$, such that $index(C^1) < index(C^2)$ because 7 is less than 10, the value of $x$ is given by $index(C^1) + 1$.

Then the computing device 100 compares at 320 $r(T_9)$ with $rer(T_9) \pm h_9$ and checks at 330 whether $rer(T_9) - h_9 < r(T_9) < rer(T_9) + h_9$. Now, the first set of predetermined conditions is satisfied: the first condition is met because $T_9$ was not affected by the failure and the second condition is met because it has already been determined (during the third iteration of the identification procedure) that $T_{10}$ was affected by the failure.

The dichotomic search algorithm may also be described by the following pseudo-code for determining the pair of indices of the reference clinical test and of the first-after-failure clinical test, e.g. 9 and 10 in the example of figure 4:

$$i = 1$$

$$j = N-1$$

$$\textit{while } i \mathrel{!=} j \textit{ do:}$$

$$R_{problem} = \underline{Decide}(i, j)$$

$$i = \textit{index of the lower limit of } R_{problem}$$

$$j = \textit{index of the upper limit of } R_{problem}$$

wherein the "subroutine" $\underline{Decide}(i, j)$ comprises the following steps, with OK indicating a test that was not affected by the failure and KO indicating a test that was affected by the failure:

$$\textit{Select } T_m, (m = \textit{integer } [(i+j)/2]) \textit{ of the range } [T_i, \ldots, T_j]$$

$$\textit{If } T_m = OK \textit{ then } R_{problem} = [T_{m+1}, \ldots, T_j]$$

$$\textit{If } T_m = KO \textit{ then } R_{problem} = [T_i, T_{m-1}]$$

$$\textit{Decide} = R_{problem}$$

Once a set of predetermined conditions is met, the method of figure 3 proceeds to step 340, wherein, in case of example B, the computing device 100 sets $T_9$ as the reference clinical test.

Going back to figure 2, after identifying the reference clinical test at 230, the computing device 100 determines at 240 a proper subset of clinical tests of the plurality of clinical tests based on the reference clinical test. For instance, the proper subset of clinical tests comprises the clinical tests that are chronologically subsequent to the reference clinical test and that have yet to be rerun.

With reference to figure 4, in example A, the clinical tests chronologically subsequent to the reference clinical test $T_7$ are $\{T_8, T_9, T_{10} T_{11}, T_{12}, T_{13}, T_{14}\}$, but $T_{14}$ has already been rerun, so that the proper subset is $\{T_8, T_9, T_{10}, T_{11}, T_{12}, T_{13}\}$. Accordingly, the proper subset is the difference between the set of chronologically subsequent clinical tests and the set of check clinical tests.

With reference to figure 4, in example B, the clinical tests chronologically subsequent to the reference clinical test $T_9$ are $\{T_{10}, T_{11}, T_{12}, T_{13}, T_{14}\}$, but $T_{10}$ and $T_{14}$ have already been rerun, so that the proper subset is $\{T_{11}, T_{12}, T_{13}\}$.

Afterwards, the computing device 100 causes at 250 the proper subset of clinical tests to be rerun. With reference to figure 4, in example A, the clinical tests $T_8, T_9, T_{10}, T_{11}, T_{12}, T_{13}$ are caused to be rerun, e.g. by the laboratory instrument 120 after the failure has been addressed, in order to obtain correct test results. With reference to figure 4, in example B, the clinical tests $T_{11}, T_{12}, T_{13}$ are caused to be rerun.

**Claims**

1.  A method comprising:

    - determining, by a computing device, a failure of a laboratory instrument configured to carry out clinical tests on biological samples;
    - accessing, by the computing device, test data comprising information specifying:

        -- a plurality of clinical tests that were carried out by the laboratory instrument and that may have been affected by the failure of the laboratory instrument,
        -- a chronological sequence of the plurality of clinical tests, and
        -- a respective plurality of test results associated with the plurality of clinical tests;

- identifying, by the computing device, a reference clinical test among the plurality of clinical tests, the reference clinical test being a clinical test that has not been affected by the failure of the laboratory instrument;
- determining, by the computing device, a proper subset of clinical tests of the plurality of clinical tests based on the reference clinical test;
- causing, by the computing device, the proper subset of clinical tests to be rerun.

2. The method of claim 1, wherein the proper subset of clinical tests comprises the clinical tests that are chronologically subsequent to the reference clinical test and that have yet to be rerun.

3. The method of claim 1 or 2, wherein identifying the reference clinical test comprises:

- performing an identification procedure for a check clinical test comprising:

-- selecting the check clinical test from the plurality of clinical tests;
-- performing a comparison between a comparison value obtained from one or more test results of the plurality of test results and one or more reference values, wherein the one or more test results of the plurality of test results comprise the test result associated with the check clinical test;
-- checking whether, based on the comparison, one or more sets of predetermined conditions are satisfied;

- when none of the sets of predetermined conditions is satisfied, repeating, by the computing device, the identification procedure for at least another check clinical test until one of the sets of predetermined conditions is satisfied;
- when one of the sets of the predetermined conditions is satisfied, setting, by the computing device, the reference clinical test based on the check clinical test.

4. The method of claim 3, wherein the one or more sets of predetermined conditions comprise one single set including a first condition that the check clinical test was not affected by the failure of the laboratory instrument.

5. The method of claim 3, wherein:

the one or more sets of predetermined conditions comprise a first set and a second set;
the first set comprises a first condition that the check clinical test was not affected by the failure of the laboratory instrument, and a second condition that a clinical test immediately chronologically following the check clinical test was affected by the failure of the laboratory instrument; and
the second set comprises a third condition that the check clinical test was affected by the failure of the laboratory instrument, and a fourth condition that a clinical test immediately chronologically preceding the check clinical test was not affected by the failure of the laboratory instrument.

6. The method of claim 4 or 5, wherein:

the one or more reference values comprise an upper limit and a lower limit;
any clinical test was not affected by the failure of the laboratory instrument when the respective comparison value is within a numerical range comprised between the lower limit and the upper limit; and
any clinical test was affected by the failure of the laboratory instrument when the respective comparison value is outside a numerical range comprised between the lower limit and the upper limit.

7. The method of any one of claims 3 to 6, wherein:
the comparison value is an exponentially weighted moving average and the exponentially weighted moving average is obtained from a subgroup of the plurality of test results including the test results associated with the check clinical test and with clinical tests chronologically preceding it.

8. The method of any one of claims 3 to 6, wherein the identification procedure for the check clinical test further comprises, after selecting the check clinical test:

causing the check clinical test to be rerun to obtain a rerun test result;
and wherein:

the comparison value is the test result associated with the check clinical test, and

the one or more reference values are obtained from the rerun test result.

9. The method of any one of the claims 3 to 8, wherein identifying the reference clinical test comprises using a dichotomic search algorithm based on the one or more sets of predetermined conditions.

10. The method of any one of the preceding claims, wherein:

accessing the test data comprises selecting the plurality of clinical tests based on one or more constraints; and the one or more constraints comprise a constraint on the plurality of test results.

11. The method of any one of the preceding claims, further comprising rerunning the proper subset of clinical tests.

12. A computing device comprising a processor configured to carry out the method according to any one of claims 1 to 10.

13. An automated laboratory system comprising the computing device according to the preceding claim.

14. A computer program product comprising instructions which, when the program is executed by a computer, cause the computer to carry out the method according to any one of claims 1 to 10.

15. A computer-readable medium comprising instructions which, when executed by a computer, cause the computer to carry out the method according to any one of claims 1 to 10.

Figure 1

**200**

```
210 ──┐  ┌─────────────────────────────────────────────┐
       └─│   Determining a failure of a laboratory instrument   │
         └─────────────────────────────────────────────┘
                              │
                              ▼
220 ──┐  ┌─────────────────────────────────────────────┐
       └─│              Accessing test data               │
         └─────────────────────────────────────────────┘
                              │
                              ▼
230 ──┐  ┌─────────────────────────────────────────────┐
       └─│          Identifying a reference clinical test         │
         └─────────────────────────────────────────────┘
                              │
                              ▼
240 ──┐  ┌─────────────────────────────────────────────┐
       └─│     Determining a proper subset of clinical tests     │
         └─────────────────────────────────────────────┘
                              │
                              ▼
250 ──┐  ┌─────────────────────────────────────────────┐
       └─│         Causing the proper subset to be rerun          │
         └─────────────────────────────────────────────┘
```

Figure 2

310 Selecting a check clinical test

320 Performing a comparison between a comparison value and reference value(s)

330 Checking whether one or more sets of predetermined conditions are satisfied

Is check at 330 affirmative?

no

yes

340 Setting the reference clinical test

230

Figure 3

Figure 4

EP 4 576 114 A1

Europäisches
Patentamt

European
Patent Office

Office européen
des brevets

## EUROPEAN SEARCH REPORT

Application Number

EP 23 29 0047

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/012191 A1 (KUCHIPUDI LAKSHMI SAMYUKTA [US] ET AL) 14 January 2016 (2016-01-14) * whole document, in particular abstract; claims; Figures 3A-3B; paragraphs [0007]-[0010], [0033]-[0093] * ----- | 1-15 | INV. G16H40/40 G01N35/00 |
| A | EP 1 413 888 A1 (A & T CORP [JP]; JAPAN PRESIDENT UNIV KOCHI) 28 April 2004 (2004-04-28) * whole document, in particular paragraphs [0004]-[0008]; claims; Figure 3 * ----- | 1-15 | |
| A | US 2010/295685 A1 (PARVIN CURTIS [US] ET AL) 25 November 2010 (2010-11-25) * whole document, in particular abstract; claims ; paragraphs [0012]-[0014] * ----- | 1-15 | |

TECHNICAL FIELDS
SEARCHED      (IPC)

G16H
G01N

The present search report has been drawn up for all claims

| Place of search | Date of completion of the search | Examiner |
|---|---|---|
| Munich | 17 May 2024 | Vanmontfort, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 29 0047

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2024

| Patent document cited in search report | | Publication date | Patent family member(s) | | Publication date |
|---|---|---|---|---|---|
| US 2016012191 | A1 | 14-01-2016 | AU | 2015203847 A1 | 28-01-2016 |
| | | | CN | 105426654 A | 23-03-2016 |
| | | | EP | 2966583 A2 | 13-01-2016 |
| | | | US | 2016012191 A1 | 14-01-2016 |
| EP 1413888 | A1 | 28-04-2004 | CN | 1529816 A | 15-09-2004 |
| | | | EP | 1413888 A1 | 28-04-2004 |
| | | | JP | 3823162 B2 | 20-09-2006 |
| | | | JP | 2003114231 A | 18-04-2003 |
| | | | KR | 20040012959 A | 11-02-2004 |
| | | | US | 2004161851 A1 | 19-08-2004 |
| | | | WO | 03012452 A1 | 13-02-2003 |
| US 2010295685 | A1 | 25-11-2010 | AU | 2010249990 A1 | 03-11-2011 |
| | | | CA | 2762415 A1 | 25-11-2010 |
| | | | CN | 102439459 A | 02-05-2012 |
| | | | EP | 2433142 A1 | 28-03-2012 |
| | | | JP | 5542201 B2 | 09-07-2014 |
| | | | JP | 2012527690 A | 08-11-2012 |
| | | | US | 2010295685 A1 | 25-11-2010 |
| | | | WO | 2010135043 A1 | 25-11-2010 |

EPO FORM P0459

For more details about this annex : see Official Journal of the European Patent Office, No. 12/82